# EUROPEAN PATENT APPLICATION

(11) **EP 0 618 224 A1**
(43) Date of publication of application: **05.10.1994**
(21) Application number: 93400824.4
(22) Date of filing: 30.03.1993
(51) Int. Cl.: C07K 5/06, A61K 37/64

(54) **Phosphonomethyldipeptides**

(71) Applicant: MERRELL DOW PHARMACEUTICALS INC., Cincinnati Ohio 45215-6300 (US)
(72) Inventor: D'Orchymont, Hugues, F-67000 Strasbourg (FR); Bigaud, Marc, F-67200 Strasbourg (FR)
(74) Representative: Gillard, Marie-Louise

(57) **Abstract**

Dipeptides and analogs thereof having a phosphonomethyl moiety attached thereto which inhibit Endothelin Converting Enzyme and are thus useful in treating conditions responsive thereto.

## Description

### Field of the Invention

This invention generally relates to dipeptides and analogs thereof having a phosphonomethyl moiety, pharmaceutical compositions comprising these compounds, processes for making such compounds, intermediates used in these processes, and methods for inhibiting the production of Endothelin. These compounds inhibit Endothelin Converting Enzyme and are thus useful in treating conditions responsive to inhibition of production of Endothelin.

### Background of the Invention

The peptide Big Endothelin, found in endothelium cells, is cleaved by the enzyme Endothelin Converting Enzyme (ECE) to produce the peptide Endothelin. Endothelin is a 21 amino acid vasoconstrictor and is produced by endothelium cells, mesangial, kidney and epithelial cells and by various human cancer cell lines and human macrophages.

Biologically, endothelin has effects on vascular smooth muscle, nonvascular smooth muscle, heart, nervous tissue, kidney and adrenal glands. Endothelin constricts arteries and veins, increases mean arterial blood pressure, decreases cardiac output, increases cardiac contractility *in vitro*, stimulates mitogenesis in vascular smooth muscle cells *in vitro*, contracts vascular smooth muscle including guinea pig trachea, human urinary bladder strips and rat uterus *in vitro*, increases airway resistance *in vivo*, induces formation of gastric ulcers, stimulates release of atrial natriuretic factor *in vitro* and *in vivo*, increases plasma levels of vasopressin, aldosterone and catecholamines, inhibits release of renin *in vitro* and stimulates release of gonadrotropin *in vitro*. See, for example, PCT patent application publication number WO 92/13545.

Possible indications for use of inhibitors of production of endothelin include treatment of cardiovascular diseases (e.g., myocardial ischemia, congestive heart failure, arrhythmia, unstable angina and hypertension); bronchoconstriction (pulmonary hypertension and asthma); neuronal action disorders (cerebral vasospasm and subarachnoid hemorrhage); endocrine disorders (preeclampsia); renal disease (acute/chronic renal failure); vascular disorders (atherosclerosis, Buergers disease, Takayasu's arteritis, Raynaud's phenomenon and complications in diabetes); cancer (especially pulmonary carcinoma; gastric mucosal damage (gastrointestinal disorders); and endotoxic shock and septicemia. See *J.Med. Chem.* 35(9): 1493-1508 (1992); *Neurology* 42: 25-31 (1992); and *Drug Development Research* 26: 361-387 (1992).

### Summary of the Present Invention

The present invention relates to a compound having the formula of : 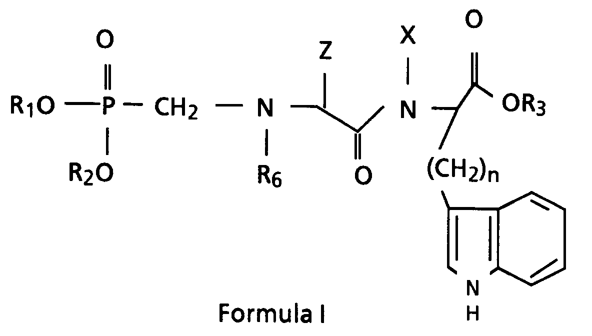
stereoisomers, hydrates, inner salts or pharmaceutically acceptable salts thereof,
wherein
R₁ or R₂ are each independently a hydrogen, C₁₋₆ alkyl, (CH₂)ₘ-aryl, R₄-C(O)O-CH(R₅)- or nothing when the inner salt is formed, provided that when one of R₁ or R₂ is a hydrogen, C₁₋₆ alkyl, or (CH₂)ₘ-aryl, then the other is hydrogen or nothing when the inner salt is formed;
R₃ is hydrogen, C₁₋₆ alkyl, (CH₂)ₘ-cycloalkyl or (CH₂)ₘ-aryl;
R₄ is C₁₋₁₀ alkyl, (CH₂)ₘ-cycloalkyl or (CH₂)ₘ-aryl;
R₅ is C₁₋₆ alkyl, (CH₂)ₘ-cycloalkyl or hydrogen;
R₆ is H, or H₂ when one of R₁ or R₂ is nothing thus forming the inner salt;
Z is (CH₂)ₘ-aryl or C₃₋₁₂ alkyl;
X is hydrogen or C₁₋₆ alkyl;
each m is independently 0, 1, 2 or 3; and
n is 1, 2 or 3.

The present invention also includes pharmaceutical compositions comprising Formula I and using the compounds of Formula I for treatment of conditions improved by the inhibition of Endothelin Converting Enzyme.

### Detailed Description of the Present Invention

As used herein, certain terms have specific meanings such as those that follow.

"Cycloalkyl" means a saturated carbon atom ring having 3 to 8 carbon atoms including, but not limited to, cyclopropyl, cyclopentyl, cyclohexyl and the like. Cycloalkyl groups can be unsubstituted or substituted with one, two or three substituents independently selected from C₁₋₆ alkyl, haloalkyl, alkoxy, thioalkoxy, amino, alkylamino, dialklyamino, hydroxy, halo, mercapto, nitro, carboxaldehyde, carboxy, alkoxycarbonyl and carboxamide.

"C₁₋₆ alkyl" means straight or branched chain alkyl moieties containing from 1 to 6 carbon atoms including, but not limited to, methyl, ethyl, n-propyl, iso-propyl, n-butyl, iso-butyl, sec-butyl, t-butyl, n-pentyl, 1-methylbutyl, 2,2-dimethylbutyl, 2-methylpentyl, 2,2-dimethylproply, n-hexyl and the like. Other numbers of carbon atoms indicated in this manner such as C₁₋₁₀ likewise represent straight or branched chain alkyls with the number of carbons indicated.

"Aryl" means monocyclic or bicyclic carbocyclic ring systems having one or more aromatic rings including, but not limited to, phenyl, naphthyl, tetrahydronaphthyl, indanyl and the like. Aryl groups can be substituted or unsubstituted with one, two or three substituents independently selected from C₁₋₆ alkyl, haloalkyl, alkoxy, thioalkoxy, aminoalkylamino, dialkylamino, hydroxy, halo, mercapto, nitro, carboxaldehyde, carboxy, carboalkoxy and carboxamide.

"Inner salt", also known as zwitterion, is a molecule which carries a positive and a negative charge, an example of which is Example 1 herein.

"Stereoisomer" is a general term for all isomers of individual molecules that differ only in the orientation of their atoms in space. It includes mirror image isomers (enantiomers), geometric (cis/trans) isomers, and isomers of compounds with more than one chiral center that are not mirror images of one another (diastereoisomers). For amino-acids, the designations L/D, or R/S can be used as described in IUPAC-IUB Joint Commission on Biochemical Nomenclature, *Eur.J.Biochem.* 138: 9-37 (1984).

"Pharmaceutically acceptable salts" means both acid addition salts and metal and amine salts which are known to be non-toxic and useful derivatives in the preparation of pharmaceutical formulations suitable for end-use applications.

Pharmaceutically acceptable acid addition salts include the known non-toxic organic or inorganic acid addition salts of the base compounds of Formula I. Illustrative organic acids which form suitable salts include the mono-, di-, and tricarboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicylic, and 2-phenoxybenzoic acids. Other organic acids which form suitable salts are the sulfonic acids such as methane sulfonic acid and 2-hydroxyethane sulfonic acid. Either the mono- or di-acid salts are prepared by standard techniques such as by dissolving the free base in an aqueous or aqueous-alcohol solution or other suitable solvent containing the appropriate acid and isolating by evaporating the solution, or by reacting the free base in an organic solvent in which case the salt separates directly or can be obtained by concentration of the solution. In general, the acid addition salts of the compounds of this invention are crystalline materials which are soluble in water and various hydrophilic forms, demonstrate higher melting points and an increased stability.

Pharmaceutically acceptable metal and amine salts are those salts which are stable under ambient conditions, and wherein the cation does not contribute significantly to the biological activity of the salt. Suitable metal salts include the sodium, potassium, calcium, barium, zinc, and aluminum salts. The sodium and potassium salts are preferred. Suitable amine salts are prepared from amines which have sufficient basicity to form a stable salt, and preferably include those amines which are frequently used in medicinal chemistry because of their low toxicity and acceptability for medical use. These include the trialkylamines such as triethylamine, and others including procaine, dibenzylamine, N-benzyl-betaphenethylamine, N-ethyl-piperidine, benzylamine, and dicyclohexylamine.

"Hydrate" is a molecule which has water in the form of H₂O molecules associated therewith.

"To Treat" or "Treatment" of a disease or condition means to prevent or alleviate the patient's disease or condition.

"Patient" refers to a warm-blooded animal, such as for example rats, mice, dogs, cats, guinea pigs, primates and humans.

The compounds of this invention may be prepared by the application of analogous chemical reactions known in the art, using reactants which are already known or which may be prepared using standard processes and techniques known in the art. In its essence, the general method for preparing the compounds of Formula I may be depicted by the reaction Schemes that follow. In those schemes, the following terms have these meanings:

"L" means leaving group. Suitable leaving groups include triflates, alkyl or aryl sulfonates (e.g, CF₃SO₂O, tosylate, mesylate) and halides (Br, Cl, or I.).

"Pg" means an appropriate protecting group. The term applies to protecting groups for atoms such as nitrogen or oxygen which are susceptible to undesirable chemical reactions. Appropriate protecting groups can be found, for example, in *Protective groups in Organic Synthesis, 2nd ed.*, Theodora W. Greene, John Wiley & Sons, Inc., New York 1991, incorporated herein by reference.

R₁, R₂, R₃, R₆, X, and Z have the meanings as previously defined. R_{1'}, R_{2'}, R_{3'} and R_{4'} are each protecting groups (Pg). It should be noted that Pg can be R₁, R₂ or R₃ except for hydrogen. 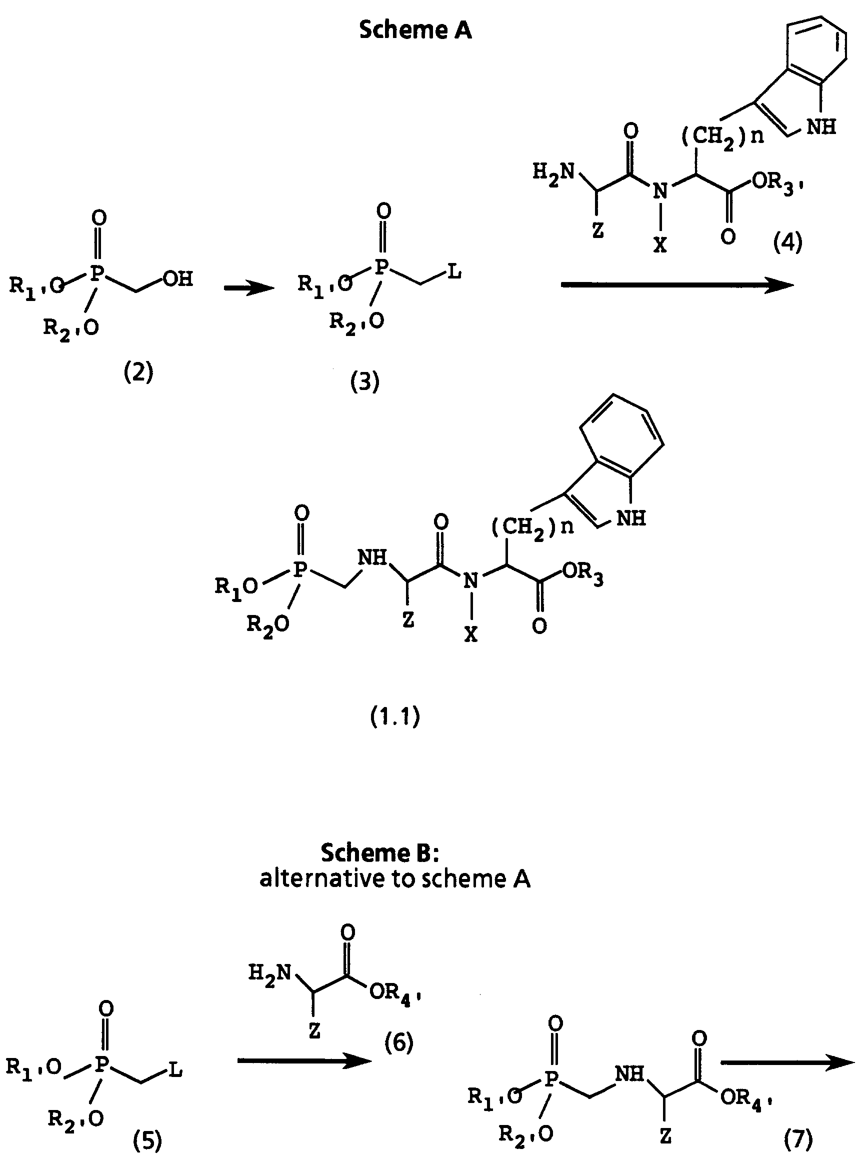 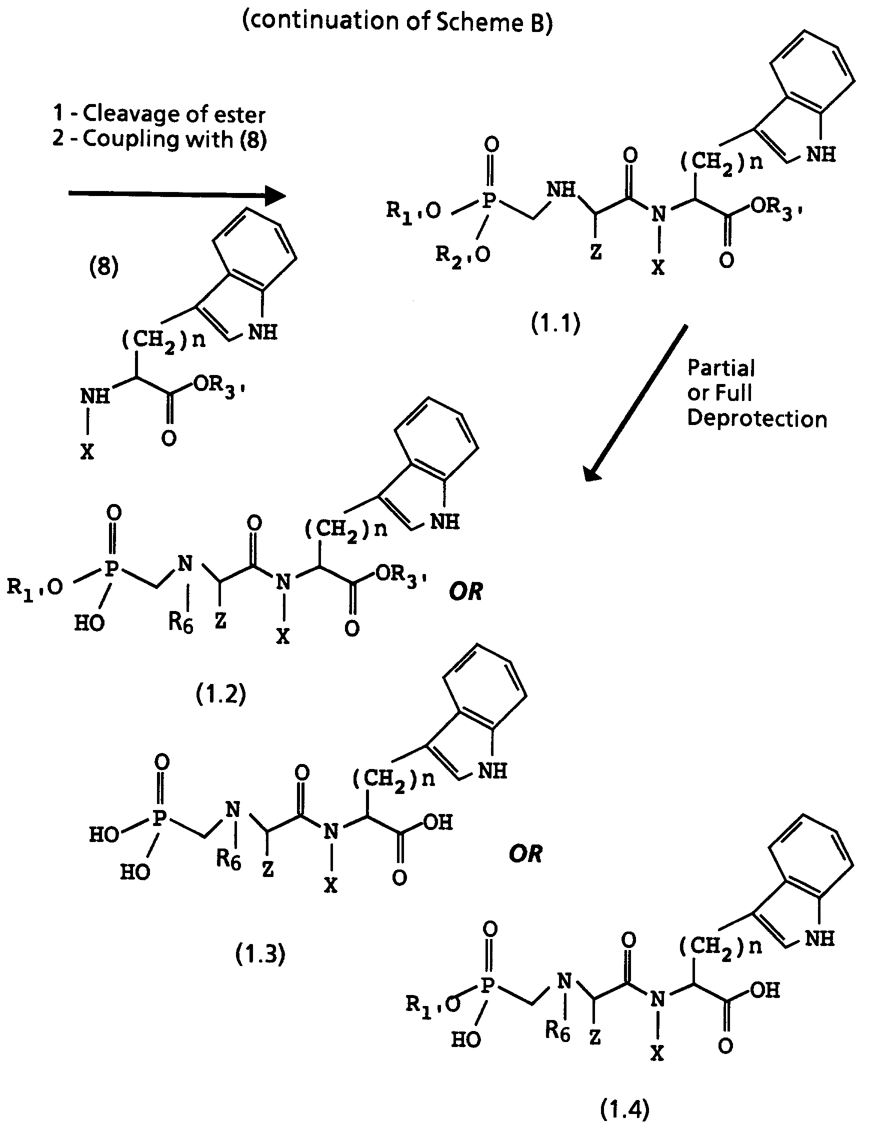
The dipeptide backbone was built using the well known strategy which consists of forming a peptide bond by coupling an aminoacid, with appropriate protecting group(s) such as tert-butyloxycarbonyl (BOC), with a peptide ester using a suitable coupling reagent. It includes the activation of carboxylate with dicyclohexylcarbodiimide (DCC) or, alternatively, according to the mixed anhydride method. Selective deprotection of the BOC to generate the free amine may be achieved using an etheral solution of hydrochloric acid, trifluoroacetic acid or formic acid. Examples of formation of the peptide bond can be found in, for example, PEPTIDE CHEMISTRY, A PRACTICAL TEXTBOOK, by Miklos Bodansky Springer-Verlag Berlin Heidelberg 1988, incorporated herein by reference. Starting materials are commercially available from, for example, Aldrich can supply dibenzylphosphite, diethylphosphite, dipropylphosphite and dibutylphosphite. Available from Bachem of Switzerland are the BOC-Leu; Leu-OMe, HCl; BOC-Phe-OH; BOC-HomoPhe-OH, BOC-D-Leu, H₂O; and Trp-OBz, HCl.

Referring to Scheme A, the introduction of the phosphonomethyl moiety utilized 1-hydroxyalkyl phosphonic esters (2) prepared by the method of A. Foucaud [*Synthesis* 916 (1982)]. The hydroxyl function is activated as a leaving group (L) (3), preferably as the trifluoromethyl sulfonate, and is substituted with the amino-terminal of a dipeptide (4) whose carboxy-terminal was protected, usually as ester shown as R_{3'}.

For example, 1-hydroxyalkyl phosphonic ester (2) is activated as the trifluoromethyl sulfonate, by reaction with trifluoromethylsulfonyl anhydride or trifluoromethylsulfonyl chloride at a low temperature ranging from -60°C to 0°C in an aprotic solvent, for example CH₂Cl₂, tetrahydrofuran, diethyl ether or dimethylformamide, or mixtures of these solvents. The protons are neutralized with a sterically hindered base, for example, 2,6-lutidine or with a metal hydride, for example, sodium hydride.

The dipeptide (4) is reacted with the activated phosphonoester (3) by adding a solution of (4) in an aprotic solvent at temperatures between O°C and 40°C for a reaction time ranging from 1 hour to 3 days. A similar base for formation of trifluoromethylsulfonate is used. The yields may be improved by adding hexamethyl phosphoramide or the like.

As an alternative shown in Scheme B, it may be also convenient to react the protected intermediate (5) with an aminoacid ester (6) to produce the phosphonomethylaminoacid ester (7). The ester (7) is de-esterified and coupled with an amino ester (8) to produce a protected phosphonomethyldipeptide (1.1).

For example, the protected intermediate (5) is reacted with the aminoacid ester (6) under similar conditions as the reaction between (3) and (4) to produce the phosphonomethylaminoacid ester (7). The ester (7) is de-esterified using a base, sodium hydroxide, potassium hydroxide or lithium hydroxide in methanol, methoxyethanol or tetrahydrofuran at room temperature for one hour to twenty-four hours.

The amino ester (8) is coupled with the de-esterified ester derived from (7) to produce the protected phosphonomethyl dipeptide (1.1) by activation of the carboxylate with dicyclohexylcarbodiimide, by forming a mixed anhydride (for example by using isobutylchloroformate) or by forming an acylazide using diphenylphosphoryl azide (DPPA). Depending on the nature of the so-formed activated ester, the reaction with the amino ester occurs between -20°C and 40°C. The acylazide reacts from -10°C to 10°C. Reaction time varies from one hour to three days.

The protected phosphono-methyldipeptide (1.1) can then be partially or fully deprotected. When all the protecting groups are benzyl, the fully deprotected compounds of Formula I (1.3) are conveniently obtained by hydrogenolysis under basic conditions in a biphasic system. Partial deprotection of the compounds of Formula I (1.2 or 1.4) may be achieved by hydrogenolysis, by action of diluted acid or base, or by reaction with trimethylsilyl halides.

The following gives specific examples of preferred compounds of the present invention and methods of making same. However, it is understood that the present invention is not to be limited by these exemplifications in any way and that other methods of making compounds known in the art may be employed.

### EXAMPLE 1

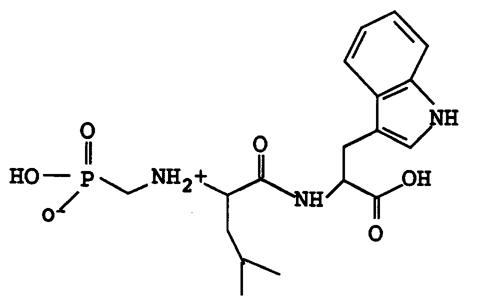

### N-(N-Phosphonomethyl-L-leucyl)-L-tryptophan

### Step A:

### N-(N-Dibenzyloxyphosphinylmethyl-L-leucyl)-L-tryptophan, benzyl ester

Dibenzyloxyphosphinylmethanol (372 mg) in anhydrous methylene chloride (5 ml) was added slowly to a methylene chloride solution (10 ml) of freshly distilled 2,6-lutidine (0.36 ml) and triflic anhydride (0.25 ml) cooled at -50°C. The mixture was stirred for 10 minutes at -50°C and the temperature was allowed to warm to 0°C for one hour.

Without isolation, to the so formed trifluoromethanesulfonic acid, dibenzyloxyphosphinylmethyl ester was then added at 0°C L-leucyl-L-tryptophan, benzyl ester (500 mg) as the free amine in anhydrous methylene chloride (5 ml). The mixture was stirred for 1 hour at 0°C and overnight at room temperature. Evaporation of the reaction mixture under vacuum gave an orange oil (1.60 g) which was purified by flash chromatography on silica gel (55 g, 230-400 Mesh). The title compound was eluted with a mixture of ethyl acetate and heptane in a ratio varying from 1/1 to 1/0. Evaporation of solvent yielded a colorless oil (240 mg). ³¹P NMR: CDCl₃ δ(relative to ext. H₃PO₄) multiplet (8 lines) (J = 9 Hz) at 27.0 ppm.

### Step B:

### N-(N-Phosphonomethyl-L-leucyl)-L-tryptophan

N-(N-Dibenzyloxyphosphinylmethyl-L-leucyl)-L-tryptophan, benzyl ester (240 mmg) and 10% palladium on charcoal (40 mg) in an heterogeneous mixture of ethyl acetate (10 ml) and aqueous solution (10 ml) of potassium hydrogenocarbonate (89 mg) were stirred at room temperature under hydrogen at normal pressure for 3 hours. The mixture was degased and the catalyst filtered off. The aqueous layer was separated, washed with ethyl acetate, filtered and lyophilized to afford a white powder (192 mg). This material was redissolved in water (10 ml) and neutralized by the slow addition of aqueous hydrochloric acid (N/10, 11 ml). The resulting precipitate was collected and dried under vacuum to afford the title compound as a white powder (115 mg).
³¹P NMR: DMSO δ(relative to ext. H₃PO₄) multiplet at 14 ppm.

| Anal. calculated for C₁₈H₂₆N₃O₆P, 1.2 H₂O | | | |
|---|---|---|---|
| | C: 49.93 | H: 6.61 | N: 9.70 |
| Found | C: 49.91 | H: 6.53 | N: 9.65 |

Melting point: endotherm at 232°C by DSC.

### EXAMPLE 2

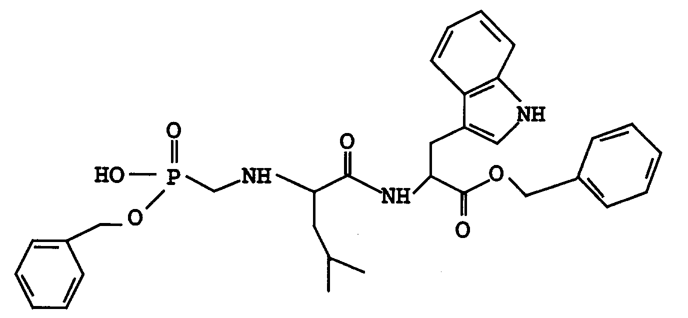

### N-(N-Benzyloxyhydroxyphosphinylmethyl-L-leucyl)-L-tryptophan, benzyl ester

N-(N-Dibenzyloxyphosphinylmethyl-L-leucyl-L-tryptophan, benzyl ester (100 mg) (Example 1, Step A) dissolved in ethyl acetate (5 ml) was hydrogenated over palladium on charcoal (10%, 15 mg) under normal pressure at room temperature for 8 hours. The mixture was filtered and the so-obtained solid was extracted with methanol to afford after evaporation of solvent the title compound as a white solid (70 mg).
³¹P NMR: CD₃OD δ(relative to ext. H₃PO₄) multiplet at 9.6 ppm.

| Anal. calculated for C₂₃H₃₈N₃O₆P, 0.5 H₂O | | | |
|---|---|---|---|
| | C: 63.99 | H: 6.54 | N: 7.00 |
| Found | C: 64.06 | H: 6.32 | N: 6.98 |

### EXAMPLE 3

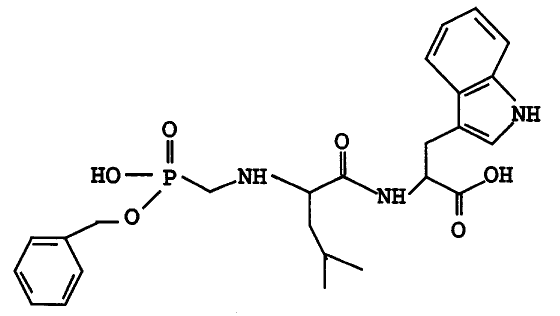

### N-(N-Benzyloxyhydroxyphosphinylmethyl-L-leucyl)-L-tryptophan

A solution N-(N-(Dibenzyloxyphosphinylmethyl-L-leucyl)-L-tryptophan, benzyl ester (100 mg) (Example 1, Step A) in ethanol (5 ml) is treated with an aqueous solution of potassium hydroxyde 0.30 ml, 1N). Stirring is maintained for 4 days at room temperature. Upon completion of the reaction the mixture is evaporated under vacuum and the residue redissolved in water and is precipitated by addition of diluted hydrochloric acid allowing the recovery of the title compound.

### EXAMPLE 4

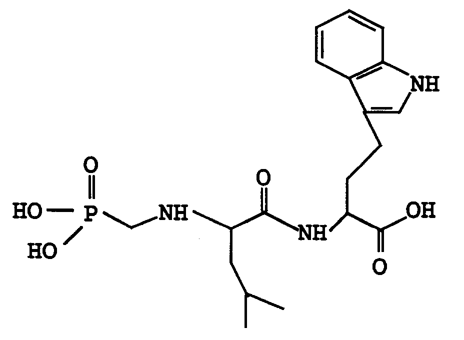

### N-(N-Phosphonomethyl-L-leucyl)-DL-homotryptophan

### Step A:

### L-Leucyl-DL-homotryptophan, ethyl ester

Ethyl 4-(β-indolyl)-2-ketobutyrate is dissolved in ethanol, then aqueous concentrated ammonia in excess and 10% Pd/C are added. The mixture is hydrogenated in a pressure apparatus at 5 bars. The catalyst is filtered and the filtrate evaporated *in vacuo* to yield a crude material which is coupled to N-tert-butyloxycarbonyl-leucine using dicyclohexylcarbodiimide with hydroxybenzotriazole in methylene chloride. Dicyclohexylurea is removed by filtration and the crude peptide obtained after evaporation of solvent is purified by chromatography on silica gel to afford N-(N-tert-butyloxycarbonyl-L-leucyl)-DL-homotryptophan, ethyl ester. Reaction with formic acid and extraction of the free base with aqueous sodium carbonate/ethyl acetate yields the title compound.

### Step B:

### N-(N-Dibenzyloxyphosphinylmethyl-L-leucyl)-DL-homotryptophan, ethyl ester

The title compound is obtained by an analogous procedure described for the preparation of N-(N-Dibenzyloxyphosphinylmethyl-L-leucyl)-L-tryptophan, benzyl ester (Example 1).

### Step C:

### N-(N-Dibenzyloxyphosphinylmethyl-L-leucyl)-DL-homotryptophan

A solution of N-(N-Dibenzyloxyphosphinylmethyl-L-leucyl)-DL-homotryptophan, ethyl ester in 2-methoxyethanol is treated with an aqueous solution of lithium hydroxyde (1 equivalent, 2-methoxyethanol/water: 9/1 in Vol.). Evaporation of solvent and acidification give the title compound.

### Step D:

### N-(N-Phosphonomethyl-L-leucyl)-DL-homotryptophan

The title compound is obtained by hydrogenation of N-(N-Dibenzyloxyphospinylmethyl-L-leucyl)-DL-homotryptophan following the procedure described for the preparation of N-(N-phosphonomethyl-L-leucyl)-L-tryptophan (Example 1).

### EXAMPLE 5

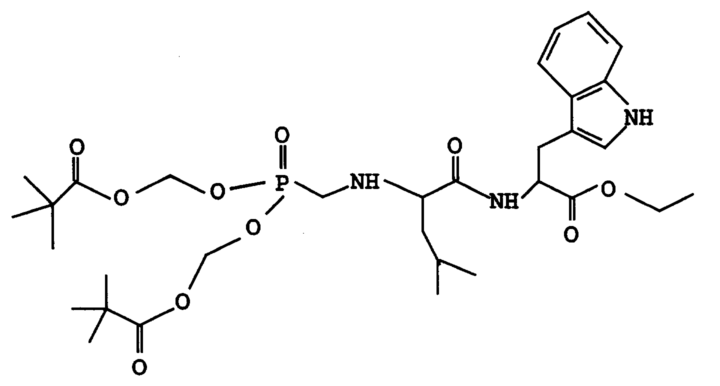

### N-[N-(di-(Pivaloyloxymethyl)-phosphonyl)-leucyl]-L-tryptophan, ethyl ester

### Step A:

### N-(N-Phosphonomethyl-L-leucyl)-L-tryptophan, ethyl ester

A solution of N-(N-Dimethylphosphonomethyl-L-leucyl)-L-tryptophan, ethyl ester is treated with trimethylsilyl bromide in methylene chloride at room temperature. The mixture is concentrated and the residue is treated with water, filtered and dried to give the title compound.

### Step B:

### N-[N-[di-(Pivaloyloxymethyl)-phosphonyl)-leucyl]-L-tryptophan, ethyl ester

A solution of N-(N-Phosphonomethyl-L-leucyl)-L-tryptophan, ethyl ester and 18 crown-6 in toluene is cooled at 0°C and is treated with potassium bis-trimethylsilylamide (2 equivalents). The mixture is reacted for 18 hours with iodomethylpivalate (2 equivalents). Ethyl acetate is added, the mixture is washed with water, dried, concentrated and chromatographed on silica gel to obtain the title compound.

### EXAMPLE 6

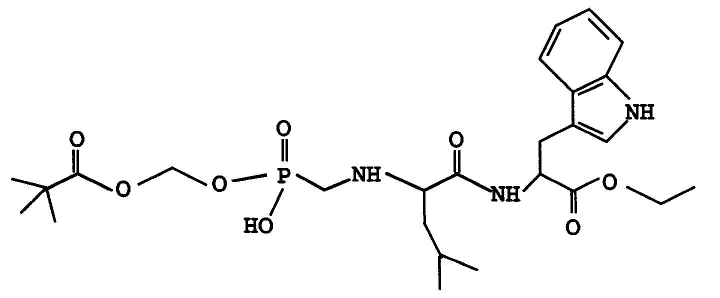

### N-(N-Pivaloyloxymethyloxyhydroxyphosphinyl-L-leucyl)-L-tryptophan, ethyl ester

A solution of N-[N-(di-Pivaloyloxymethyl)-phosphonyl)-leucyl]-L-tryptophan, ethyl ester in ethanol is cooled at 0°C and sodium hydroxyde (1 equivalent) is added dropwise. Stirring is continued for 15 minutes and the reaction mixture is neutralized with 1N hydrochloric acid (1 equivalent). The mixture is concentrated under reduced pressure, the residue is partioned between methylene chloride and cold water. The organic layer is dried and concentrated under vacuum. The phosphonic acid monoester is finally purified by flash chromatography on silica gel.

### EXAMPLE 7

In order to show the efficacy of the present compounds as antihypertensive agent, the following protocol may be used.

Male, Sprague-Dawley rats (250-300 g) are anaesthetized with urethane (1.25 g/kg, i.p.), supplemented as required. Tygon catheters are inserted into the right main carotid artery and the right femoral vein for arterial pressure monitoring and intravenous drug injections respectively. The preparation is allowed to stabilize for 30 minutes before starting the experimental protocol. Arterial pressure is monitored continuously with a TA2000 Gould stripchart recorder coupled to a data acquisition system (Dataflow from Crystal Biotech, U.S.A.).

Rats are randomized to be pretreated with an i.v. bolus injection (100 µl/100 g, flushed with 0.3 ml saline) of either saline or a dose (ranging from 1 to 100 µmol/kg) of phosphoramidon, or the compounds of the present invention. Five minutes later, Pro-ETl (1 nmol/kg) is administered as an intravenous bolus injection (10 µl/100 g, flushed with 0.3 ml saline) and changes on arterial pressure are recorded for 30 minutes. To avoid any artefact, each rat receives only one pretreatment and only one dose of Pro-ETl. Pro-ET-l elicits the hypertensive response, and phosphoramidon inhibits same. The compounds of the present invention are measured against the inhibition of phosphoramidon.

Human/porcine Pro-ETl may be obtained from Peptide Institute (Osaka, Japan) and dissolved in 0.1% acetic acid to give 10⁻⁴M stock solutions, whose exact concentrations can be checked by absorbance spectrophotometry at 280 nm using an extinction coefficient of 7245M⁻¹cm⁻¹. The stock solutions are then aliquoted, lyophilized and stored at - 20°C. The peptide is redissolved and diluted in saline on the day of the experiment.

### EXAMPLE 8

In order to show the efficacy of the compounds of the present invention in the treatment of subarachnoid hemorrhage, the procedure used in *Life Sciences* 49: 841-848 (1991) may be followed.

### EXAMPLE 9

In order to show the efficacy of the compounds of the present invention in the treatment of asthma, Hartley guinea pigs of either sex are passively sensitized by intracardiac injection of antiserum prepared in rabbits (IgC) or guinea pigs (IgG or IgE) against a specific antigen (ovalbumin). The compounds are administered to the sensitized guinea pigs with route, dose, and time of compound administration dependent upon experimental design. The guinea pigs are challenged by aerosolization of the antigenic solution. The time to prostration following aerosolization and the incidence of mortality after five minutes are recorded. Negative controls consist of passively sensitized guinea pigs receiving no treatment and the incidence of lethality in this group is generally 90 to 100%. Chlorpheniramine and cyproheptadine may serve as positive controls.

### EXAMPLE 10

In order to show the efficacy of the compounds of the present invention in the treatment of congestive heart failure, the procedure used in *Circulation* 82(6); 2226-2230 (1990) may be followed. This procedure is modified by pretreating the test animal with the compounds of the present invention.

### EXAMPLE 11

In order to show the efficacy of the compound of the present invention in the treatment of renal failure, the procedure used in *European Journal of Pharmacology* 221: 77-83 (1992) may be followed.

For pharmacological end-use applications, the compounds of Formula I are preferentially administered in the form of their pharmaceutically acceptable acid addition salts. Of course, the effective dosage of the compounds will vary according to the indication for use, individual potency of each compound employed, the severity and nature of the disease being treated and the particular subject being treated. In general, effective results can be achieved by administering a compound at a dosage of about 0.01 mg to about 20 mg per kilogram of body weight per day, administered systemically. Therapy should be initiated at lower dosages. The dosage thereafter may be administered orally in solid dosage forms, e.g., capsules, tablets, or powders, or in liquid forms, e.g., solutions or suspensions. The compounds may also be injected parenterally in the form of sterile solutions or suspensions.

In practicing the method of this invention, the active ingredient is preferably incorporated in a composition comprising a pharmaceutical carrier and from about 5 to about 90 percent by weight of a compound of the invention or a pharmaceutically-acceptable salt thereof. The term "pharmaceutical carrier" refers to known pharmaceutical excipients useful in formulating pharmaceutically active compounds for internal administration to animals, and which are substantially non-toxic and non-sensitizing under conditions of use. The compositions can be prepared by known techniques for the preparation of tablets, capsules, elixirs, syrups, emulsions, dispersions and wettable and effervescent powders, and can contain suitable excipients known to be useful in the preparation of the particular type of composition desired.

The preferred route of administration is oral administration. For oral administration the formula I compounds can be formulated into solid or liquid preparations such as capsules, pills, tablets, troches, lozenges, melts, powders, solutions, suspensions, or emulsions. The solid unit dosage forms can be a capsule which can be of the ordinary hard- or soft-shelled gelatin type containing, for example, surfactants, lubricants, and inert fillers such as lactose, sucrose, calcium phosphate, and cornstarch. In another embodiment the compounds of this invention can be tableted with conventional tablet bases such as lactose, sucrose, and cornstarch in combination with binders such as acacia, cornstarch, or gelatin, disintegrating agents intended to assist the break-up and dissolution of the tablet following administration such as potato starch, alginic acid, corn starch, and guar gum, lubricants intended to improve the flow of tablet granulations and to prevent the adhesion of tablet material to the surfaces of the tablet dies and punches, for example, talc, stearic acid, or magnesium, calcium, or zinc stearate, dyes, coloring agents, and flavoring agents intended to enhance the aesthetic qualities of the tablets and make them more acceptable to the patient. Suitable excipients for use in oral liquid dosage forms include diluents such as water and alcohols, for example, ethanol, benzyl alcohol, and the polyethylene alcohols, either with or without the addition of a pharmaceutically acceptable surfactant, suspending agent, or emulsifying agent.

The formula I compounds of this invention may also be administered parenterally, that is, subcutaneously, intravenously, intramuscularly, or interperitoneally, as injectable dosages of the compound in a physiologically acceptable diluent with a pharmaceutical carrier which can be a sterile liquid or mixture of liquids such as water, saline, aqueous dextrose and related sugar solutions, an alcohol such as ethanol, isopropanol, or hexadecyl alcohol, glycols such as propylene glycol or polyethylene glycol, glycerol ketals such as 2,2-dimethyl-1,3-dioxolane-4-methanol, ethers such as polyethylene glycol 400, an oil, a fatty acid, a fatty acid ester or glyceride, or an acetylated fatty acid glyceride with or without the addition of a pharmaceutically acceptable surfactant such as a soap or a detergent, suspending agent such as pectin, carbomers, methylcellulose, hydroxypropylmethylcellulose, or carboxymethylcellulose, or emulsifying agent and other pharmaceutically acceptable adjuvants. Illustrative of oils which can be used in the parenteral formulations of this invention are those of petroleum, animal, vegetable, or synthetic origin, for example, peanut oil, soybean oil, sesame oil, cottonseed oil, corn oil, olive oil, petrolatum, and mineral oil. Suitable fatty acids include oleic acid, stearic acid, and isostearic acid. Suitable fatty acid esters are, for example, ethyl oleate and isopropyl myristate. Suitable soaps include fatty alkali metal, ammonium, and triethanolamine salts and suitable detergents include cationic detergents, for example, dimethyl dialkyl ammonium halides, alkyl pyridinium halides; anionic detergents, for example, alkyl, aryl, and olefin sulfonates, alkyl, olefin, ether, and monoglyceride sulfates, and sulfosuccinates; nonionic detergents, for example, fatty amine oxides, fatty acid alkanolamides, and polyoxyethylenepolypropylene copolymers; and amphoteric detergents, for example, alkyl beta-aminopropionates, and 2-alkylimidazoline quarternary ammonium salts, as well as mixtures. The parenteral compositions of this invention will typically contain from about 0.5 to about 25% by weight of the formula I compound in solution. Preservatives and buffers may also be used advantageously. In order to minimize or eliminate irritation at the site of injection, such compositions may contain a non-ionic surfactant having a hydrophile-lipophile balance (HLB) of from about 12 to about 17. The quantity of surfactant in such formulations ranges from about 5 to about 15% by weight. The surfactant can be a single component having the above HLB or can be a mixture of two or more components having the desired HLB. Illustrative of surfactants used in parenteral formulations are the class of polyethylene sorbitan fatty acid esters, for example, sorbitan monooleate and the high molecular weight adducts of ethylene oxide with a hydrophobic base, formed by the condensation of propylene oxide with propylene glycol.

The compounds of this invention can also be administered topically. This can be accomplished by simply preparing a solution of the compound to be administered, preferably using a solvent known to promote transdermal absorption such as ethanol or dimethyl sulfoxide (DMSO) with or without other excipients. Preferably topical administration will be accomplished using a patch either of the reservoir and porous membrane type or of a solid matrix variety.

Some suitable transdermal devices are described in U.S. Patent Nos. 3,742,951, 3,797,494, 3,996,934, and 4,031,894. These devices generally contain a backing member which defines one of its face surfaces, an active agent permeable adhesive layer defining the other face surface and at least one reservoir containing the active agent interposed between the face surfaces. Alternatively, the active agent may be contained in a plurality of microcapsules distributed throughout the permeable adhesive layer. In either case, the active agent is delivered continuously from the reservoir or microcapsules through a membrane into the active agent permeable adhesive, which is in contact with the skin or mucosa of the recipient. If the active agent is absorbed through the skin, a controlled and predetermined flow of the active agent is administered to the recipient. In the case of microcapsules, the encapsulating agent may also function as the membrane.

In another device for transdermally administering the compounds in accordance with the present invention, the pharmaceutically active compound is contained in a matrix from which it is delivered in the desired gradual, constant and controlled rate. The matrix is permeable to the release of the compound through diffusion or microporous flow. The release is rate controlling. Such a system, which requires no membrane is described in U.S. Patent No. 3,921,636. At least two types of release are possible in these systems. Release by diffusion occurs when the matrix is non-porous. The pharmaceutically effective compound dissolves in and diffuses through the matrix itself. Release by microporous flow occurs when the pharmaceutically effective compound is transported through a liquid phase in the pores of the matrix.

The compounds of the present invention may be incorporated into an aerosol preparation by means commonly known to those skilled in the art. The aerosol preparation may be prepared for use as a topical aerosol or may be prepared for inhalation. The aerosol preparation may be in the form of a solution or suspension and may contain other ingredients such as solvents, propellants and/or dispersing agents. Typical examples of aerosol preparations are shown in *Remington's Pharmaceutical Sciences, 18th ed.*, Mack Publishing Company, Easton Pennsylvania, pp. 1694-1712 (1990).

As is true for most classes of compounds suitable for use as therapeutic agents, certain subgeneric groups and certain specific compounds are preferred. In this instance those compounds which are preferred have R₁ as H, R₂ as nothing and R₆ as H₂, R₃ as H or (CH₂) aryl, Z as iso-butyl, and n as 1.

## Claims

1. A compound having the formula of: 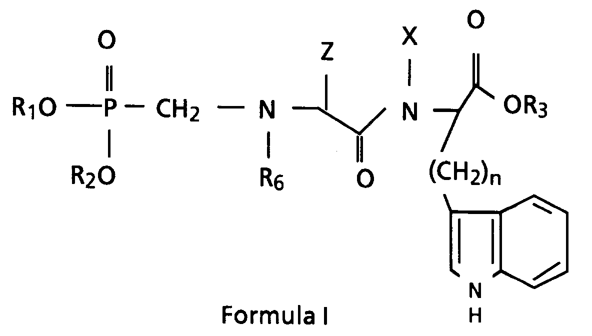 stereoisomers, hydrates, inner salts or pharmaceutically acceptable salts thereof,
wherein
R₁ or R₂ are each independently a hydrogen, C₁₋₆ alkyl, (CH₂)ₘ-aryl, R₄-C(O)O-CH(R₅)- or nothing when the inner salt is formed, provided that when one of R₁ or R₂ is a hydrogen, C₁₋₆ alkyl, or (CH₂)ₘ-aryl, then the other is hydrogen or nothing when the inner salt is formed;
R₃ is hydrogen, C₁₋₆ alkyl, (CH₂)ₘ-cycloalkyl or (CH₂)ₘ-aryl;
R₄ is C₁₋₁₀ alkyl, (CH₂)ₘ-cycloalkyl or (CH₂)ₘ-aryl;
R₅ is C₁₋₆ alkyl, (CH₂)ₘ-cycloalkyl or hydrogen;
R₆ is H, or H₂ when one of R₁ or R₂ is nothing thus forming the inner salt;
Z is (CH₂)ₘ-aryl or C₃₋₁₂ alkyl;
X is hydrogen or C₁₋₆ alkyl;
each m is independently 0, 1, 2 or 3; and
n is 1, 2 or 3.

2. The compound of claim 1 wherein R₁ is H.

3. The compound of claim 1 wherein R₂ is nothing and R₆ is H₂.

4. The compound of claim 1 wherein R₃ is H or (CH₂)-aryl.

5. The compound of claim 1 wherein Z is iso-butyl.

6. The compound of claim 1 wherein X is H.

7. The compound of claim 1 wherein the compound is selected from a group consisting of
N-(N-Phosphonomethyl-L-leucyl)-L-tryptophan,
N-(N-Benzyloxyhydroxyphosphinylmethyl-L-leucyl)-L-tryptophan, benzyl ester,
N-(N-Benzyloxyhydroxyphosphinylmethyl-L-leucyl)-L-tryptophan,
N-(N-Phosphonomethyl-L-leucyl)-DL-homotryptophan,
N-[N-(di-(Pivaloyloxymethyl)-phosphonyl)-leucyl]-L-tryptophan, ethyl ester, and
N-(N-Pivaloyloxymethyloxyhydroxyphosphinyl-L-leucyl)-L-tryptophan, ethyl ester.

8. A compound according to claim 1 for use as a pharmaceutically active compound.

9. A pharmaceutical composition according to claim 1, optionally in combination with a pharmaceutically acceptable carrier.

10. A compound according to claim 1 for use in the treatment of cardiovascular diseases, bronchoconstriction, neuronal action disorders, vascular disorders and renal diseases, which are conditions responsive to inhibition of endothelium.

11. Use of a compound according to claim 1, optionally in combination with a pharmaceutically acceptable carrier, for the preparation of a pharmaceutical composition for the treatment of cardiovascular diseases, bronchoconstriction, neuronal action disorders, vascular disorders and renal diseases, which are responsive to inhibition of endothelium.
